# EUROPEAN PATENT SPECIFICATION

(11) **EP 3 059 612 B1**
(45) Date of publication and mention of the grant of the patent: **13.06.2018**
(21) Application number: 16156796.1
(22) Date of filing: 23.02.2016
(51) Int. Cl.: G01T 1/04, A61N 5/10

(54) **3D DOSIMETER AND DOSIMETRIC METHOD USING 3D DOSIMETER**
3D DOSIMETER UND DOSIMETRIE-VERFAHREN MIT 3D DOSIMETER
3D DOSIMÈTRE ET LE PROCÉDÉ DE DOSIMÉTRIE UTILISANT LE 3D DOSIMÈTRE

(30) Priority: 23.02.2015 IT UB20150449
(43) Date of publication of application: 24.08.2016
(73) Proprietor: Universita degli Studi della Calabria, 87036 Rende (IT); Universita'Degli Studi di Trieste, I-34127 Trieste (IT)
(72) Inventor: Sportelli, Luigi, 87036 Rende (IT); Marsich, Eleonora, 34100 Trieste (IT); Santaniello, Anna, 87037 San Fili CS (IT)
(74) Representative: Perrotta, Aldo

(56) References cited:
- JP-A- H02 173 589
- US-A1- 2003 099 571
- GUIDELLI EDER JOSÉ ET AL: "Influence of photon beam energy on the dose enhancement factor caused by gold and silver nanoparticles: An experimental approach", MEDICAL PHYSICS, AIP, MELVILLE, NY, US, vol. 41, no. 3, 1 January 1901 (1901-01-01), XP012182233, ISSN: 0094-2405, DOI: 10.1118/1.4865809 [retrieved on 1901-01-01]
- PEIMEL-STUGLIK Z ET AL: "EPR spectra of gamma-irradiated dl-alpha-alanine supported on molecular sieves", SPECTROCHIMICA ACTA. PART A: MOLECULAR AND BIOMOLECULAR SPECTROSCOPY, ELSEVIER, AMSTERDAM, NL, vol. 69, no. 5, 1 May 2008 (2008-05-01), pages 1395-1404, XP022615564, ISSN: 1386-1425, DOI: 10.1016/J.SAA.2007.09.042 [retrieved on 2008-04-17]

## Description

### TECHNOLOGICAL FIELD

The present invention relates to a 3D dosimeter that uses a newly developed material and to a method for using such a device for the verification of the dose delivered in radiotherapeutic medical applications and for quality controls of the radiation beams.

### STATE OF THE ART

The verifications of spatial distribution of the values of the radiation dose scheduled for the therapeutic treatment and the quality controls of the radiation beam are part of the mandatory measuring procedures for the therapeutic use of radiation beams. The object is to maintain the uncertainties of the dose delivered to the patient within the limits agreed by the clinical community (typically under 5%), in order to achieve an effective therapy, while maintaining the side effects as low as possible. Measurements of beam parameters and dose are made by using appropriate dosimetric devices, according to procedures agreed by the medical community and established within clinical protocols.

Innovative radiotherapeutic techniques in clinical use in recent years aim to optimize the radiative treatment of cancer by providing the maximum dose to the diseased region and limiting as much as possible the damage to surrounding healthy tissue. Compared to traditional methods, the particular set-up used in the treatment of a given patient is characterized, for a certain distance source-patient, by a beam profile modulated in term of intensity, and a function of the direction of radiation. In this way, the delivered dose reflects the three-dimensional (3D) distribution of the tumour tissue.

The current measuring devices of the dose are not always adequate to meet these specific 3D requirements. Many types of dosimeters performs "point" dose detections, that is, in a limited volume. In the case of the electron spin magnetic resonance dosimetry, for example, tablets are used that are formed by pressing powders, with a minimum linear size of the order of a few mm, and a maximum size up to about 1 cm. The ionization chambers, which are among the most used clinical dosimeters, has also similar features. If it is necessary to acquire the information in a large region, it is necessary to replace the dosimeter in a different position and to repeat the reading, or to arrange initially a plurality of dosimeters within the region of interest. This involves long measurement sessions, and increases operating costs. The extent of human and material resources involved in the verification procedure or in the verification of the delivered dose, having in mind the oncological patient's urgent needs, often hampers the use of the new radiotherapeutic technique. A 3D dosimeter, enabling a rapid performing of the measure, promotes the spread of the innovative radiotherapy scheme.

The most widespread 3D dosimeters are polymer gels: the polymerization is radio-induced in a volume of several centimetres, i.e. a size comparable to that of the tumour and the surrounding healthy tissues as a whole. The effect is revealed by 3D imaging techniques (computed tomography, nuclear magnetic resonance imaging). However, these gels are toxic. In addition, the progress of the polymerization reaction after irradiation, despite is very slow in the more recent gel composition formulations, leads to an alteration of the image, which represents a loss of accuracy in the measurement of dose. These aspects are reviewed for example by C. Baldock et al., Polymer gel dosimetry, Physics in Medicine and Biology, 55 (2010) R1-R63.

The 3D dosimeter of the invention is a biocompatible material. Indeed, it is currently used in the osteoimplant field. The signal is acquired during a single exposure in a different area of several centimetres, has a 3D nature, and is stable. The average density of the material that constitutes the dosimeter can be varied in a range of values comparable with those of low-density biological tissues, such as lung.

The tumour dose simulated in a treatment plan is calculated at particle equilibrium conditions. In such circumstances, in fact, it is proportional to calculable physical amounts. Under particles equilibrium conditions, the electronic transport phenomena are similar in materials having similar densities. The dosimeters are therefore manufactured with materials having a density comparable to that of the diseased organ (tissue equivalence). This avoids the uncertainties arising from the use of correction factors in the the calculated value-measured value comparison. The particle equilibrium conditions are often not met in low-density biological tissues, especially for high-energy radiations and innovative radiotherapeutic techniques, as illustrated in detail by recent studies by B. Disher et al., Phys. Med. Biol. 57 (2012) 1543-1559. Among these tissues, the pulmonary tissue has an average density of about 1/3 and 1/10 of that of the muscle tissue, for the healthy and the emphysematous tissue, respectively. By contrast, the carcinogenic formations have a density close to 1. The differences between delivered, measured and calculated dose are particularly significant in the radiotherapy of the lung. A similar situation occurs for other low-density materials, such as the tissues in the head and neck sites.

Consider by way of example the lung cancer. The pulmonary neoplastic diseases are among the leading causes of death from cancer. In the male population, it is already the leading cause of death, and it is estimated that within the next two years will exceed the breast cancer as a cause of mortality in women. The dose delivered to the lung may be drastically different from the planned treatment (up to 80%), resulting in a reduced therapeutic efficacy for cancer and/or an increase of radioprotection risk to the surrounding healthy tissues. In addition, the assessment of the received dose will be affected when the lung is not the target of the treatment but an organ at risk because of the presence of tumours in nearby organs, such as in the case of radiotherapy to the breast or head and neck cancer.

Therefore, dosimetric devices, which are more appropriate to the specific characteristics of lung tissue and other low-density biological tissues, are desired by the clinical community both in order to assist the tissue radiotherapeutic treatments and to evaluate its effective exposure as healthy tissue.

The following document is also acknowledged herein: Guidelli Eder Jose et al "Influence of photon beam energy on the dose enhancement factor caused by gold and silver nanoparticles: An experimental approach", MEDICAL PHYSICS, AIP, MELVILLE, NY, US, vol. 41, no. 3, March 2014.

### BRIEF DESCRIPTION OF THE INVENTION

The invention is defined in the appended set of claims. Subject of this invention is a 3D dosimeter, which allows a three-dimensional mapping of the radiation dose distribution in a single exposure.

The invention involves the inventive use of a hydrogel composite that presents radiosensitivity through the reading of the electron spin magnetic resonance signal (EPR). The material has been recently synthesized for tissue engineering applications. It is proposed for the first time for use in the physical dosimetry of radiation beams.

Normal sensitive EPR materials are powders. They allow accurate determinations of point doses. The material proposed herein is an extended and self-supporting material allowing a 3D recording of the radiation dose.

The hydrogel is in fact characterized by a porous morphology similar, for example, to the lung morphology, and can have an average density close to those of various low-density biological tissues.
(1) The dosimetric material is constituted by a microporous polymeric matrix (alginate) that incorporates nanometric microporous crystals of inorganic material (hydroxyapatite). Alginates are natural polysaccharides that form stable gels when added with divalent atoms such as calcium. The latter is contained in hydroxyapatite crystals.

The use of the hydrogel for 3D dosimetry, proposed in this invention, is based on its radiation-sensitivity properties. The effect of the radiation is detected by means of a magnetic electron spin resonance spectroscopy (EPR) measurement, as described in detail below. The hydrogel can be used as a material for the electron spin magnetic resonance dosimetry (EPR dosimetry) since the measured signal is due to radiation and is stable. (2) The EPR dosimetry is a physical method, which uses the electron spin magnetic resonance to determine the energy absorbed per mass unit (the dose) by a suitable radiosensitive material irradiated with beams of ionizing particles. The number of radio-induced unpaired electron spins (free radicals) in the material is due to the dose through a calibration procedure.

The inventive use of the material can exploit its unique density and morphology characteristics. Average density and pore size of the material can be varied in a controlled manner within a range of values, such as to reproduce the characteristics of the different zones of the lung, of the low-density biological tissues present in other body areas (for example in the head and neck), and also of the pathological emphysematous pulmonary tissue (lower density to the healthy tissue) or the carcinogenic tissue (higher density to healthy tissue).

In the detailed description of the invention, dosimetric characteristics of the material and the manner by which it can be used as a 3D dosimeter will be illustrated.

### BRIEF DESCRIPTION OF DRAWINGS

In the following detailed description, reference will be made to the accompanying drawings, in which:
Fig. 1a shows a photo of two cylindrical dosimeters made with the material, realized with linear dimensions of about 1 cm and several centimetres. The density of the small dosimeter is 0.3 g/cm³, while that of the large dosimeter is 0.1 g/cm³.
Fig. 1b shows the SEM image of the pore walls at the maximum magnification. The inorganic material crystals immersed in the organic matrix can be seen.
Fig. 1c shows the SEM image of the material at a magnification comparable with that of Fig. 1a is started. The interconnected pores, separated by thin walls of material, are visible.
Fig. 1d shows the morphology of the lung parenchyma (from literature) as it appears in the examination by scanning electron microscopy (SEM). Alveolar cavities are distinguishable.
Fig. 2 shows the schematic design of a heterogeneous dosimeter, composed so as to accommodate therein elements having different density, for example a lump of cancerous cells.

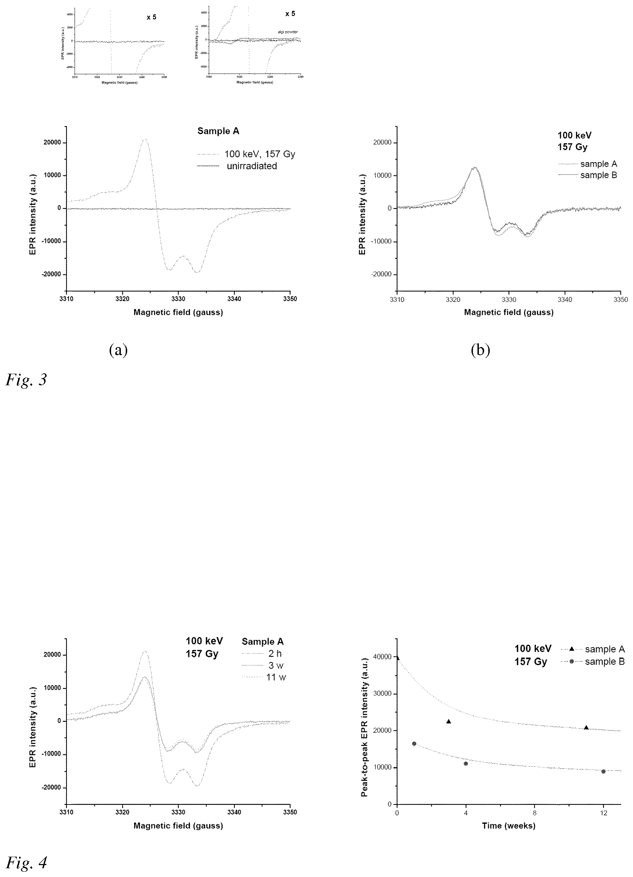

Fig. 3a shows the comparison between the EPR signals from a sample of the material (about 100 mg) before and after exposure to a beam of X rays with peak energy of 100 keV (radiating device model Faxitron RX-650, inherent filtration 1.6 mm) at a distance of 50 cm for 59 minutes, as it appears when it is measured 2 hours after the irradiation. The exposure rate is 266 Roentgen/min, corresponding to a nominal dose of 157 Gy in water. The magnifications in the insets show the absence of free radicals in the magnetic field region of interest before the irradiation of the of the material, and after the irradiation of a powder sample of the organic matrix of the material.
Fig. 3b shows the comparison between the stable EPR signals obtained for two samples at density 0.3 g/cm³ under the same irradiation conditions. The spectra are normalized to the maximum value. Note the presence of a weak spectral contribution due to impurities in one of the two samples (A). The measures were carried out approximately three months after the irradiation.
Fig. 4a shows the time evolution of the EPR signal during the first three months irradiation (sample A).
Fig. 4b shows the decay of the peak-peak amplitude of the EPR signal of two different samples irradiated under the same conditions. The signal is normalized to the mass of the material.

### DETAILED DESCRIPTION OF THE INVENTION

Unlike existing EPR dosimeters, consist of dust possibly pressed to form tablets, and therefore suitable to accurate determinations of the dose, the dosimeters of the invention are extended, self-supported, and inherently suitable for three-dimensional measurements of the dose.

They allow the acquisition of information of the dose at the same time in a wide spatial region, and store the information for the next reading.

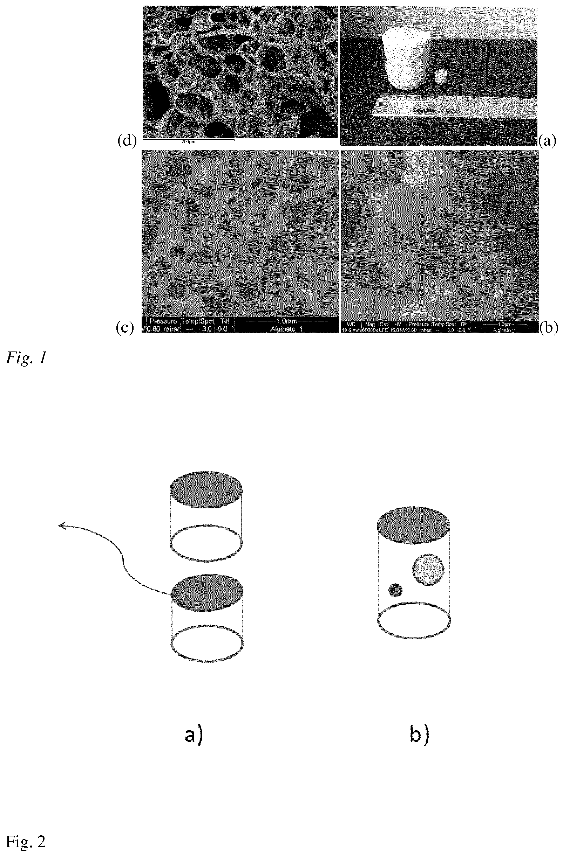

A photo of two 3D dosimeters of hydroxyapatite alginate (HAPA) is shown in Fig. 1a is started. In the embodiments shown, the dosimeters are cylindrical in shape and have linear dimensions (diameter and height) of about 1 cm or a few cm. Their densities are 0.3 g/cm³ and 0.1 g/cm³, respectively. They were manufactured following the processes known to the skilled in the art, which are therefore not described in detail here.

Alginates are natural polymers obtained from the extracellular matrix of the brown seaweed, made up of polysaccharide chains. They form stable hydrogels in the presence of divalent atoms such as Ca. The hydrogel HApA is a composite material for the first time synthesized in the last decade, and (3) it has been intensively studied for its properties of interaction with tissue constituents, which are interesting for the realization of biocompatible scaffolds for endosseous dental implants. It is obtained starting from a solution of the organic component (alginate) with the inorganic component (hydroxyapatite crystals, Ca₅(PO₄)₃(OH)). In solution, the hydroxyapatite crystals provide Ca atoms necessary for the formation of the gel (G. Turco et al., et al., Biomacromolecules, 2009, 10: 1575-1583).

The crystals used for the growth of the hydrogel have nanometric dimensions (width of the order of tens of nm and length of the order of 100 nm). These crystals, embedded in the walls that delimit the dell'HApA pores, can be seen in Fig. 1b.

The hydrogel pores are interconnected, and have typical dimensions of the order of hundreds of microns in the small dosimeter in Fig. 1c, comparable with the section of the pulmonary alveoli, as reported in literature, for example, A.D. Freed et al., PNNL-21287, US Department of Energy, March 2012, shown in Fig. 1d. The size of the pores and the density of the material depend on the proportions between the organic and inorganic components, and on the preparation procedure, as shown for example by A. Tampieri et al., Acta Biomater. 1 (2005) 343-351. In our dosimeters about 95% is alginate, and then the hydroxyapatite is a very small fraction, of the order of a few percentage points.

The 3D dosimeters can be obtained with different shapes and sizes (see Fig. 1). The dosimeter can also be composed of hydrogel with portions of different density, as schematically shown in Fig. 2, in order to reproduce the spatial distribution of healthy and diseased tissues. The heterogeneity of physiological and pathological nature is an organ inherent source of uncertainty in the delivery of the dose. The reproducing of it in the dosimeter aims to achieve a better match between the delivered dose value and the measured value during the dose verification phase of the treatment plan, or during the phase of beam quality control.

The radiosensitivity of the hydrogel for dose values of interest in clinical settings is shown in Fig. 3. The measurement is obtained by using a portion of the small dosimeter in Fig. 1, irradiated with a beam of photons having an energy in the range of the low energies of medical interest (orthovoltage). The mass of material, of about 100 mg, is pulverized, and then inserted in the tube for the EPR measurement. The measurement is obtained by the spectrometer Bruker ESP300 in X-band.

The electron spin magnetic resonance spectroscopy technology reveals the unpaired electron's spin states induced in the interaction between the incident ionizing radiation beam and the atomic constituents of the material.

There are no spins in the region of the free radicals before the irradiation, as shown in Fig. 3a, left inset.

The radio-induced spin states in the organic component of the hydrogel is negligible, as clear from the Fig. 3a, right inset.

Fig. 3a demonstrates, in conclusion, that the alginate component is EPR-silent, that the EPR signal is radio-induced, and that it comes exclusively from hydroxyapatite crystals.

The spectra of the composite HApA of Fig. 3 appear very similar to those of the single phase of hydroxyapatite available in the literature, see, for example, P. Fattibene and F, Callens, Appl. Radiat. Isot. 68 (2010) 2033-2116.

In short, the spectrum measured in the first derivative in X band consists of a separate positive peak by a double negative peak of +4.3 and +9.3 gauss, respectively. (6) These structures are originated by the superposition of unpaired pin states with the same chemical-physical nature, but placed in two environments with different local symmetries: one with a main orthorhombic symmetry, and one with a small contribution of isotropic symmetry. The spins observed were associated with the free radical states CO₂⁻ radio-induced in the CO₂ impurities of the hydroxyapatite crystal.

Free radicals of other nature, associated with chemically different impurities, may contribute to a lesser extent to the spectrum at lower values of magnetic field. They correspond to a wide structure having a low intensity centred at about -7 gauss from the main peak. This structure is distinguishable only in some of our samples, as shown in Fig. 3b.

The intensity of the EPR peaks depends on the preparation, as shown in Fig. 3b. This is due to the different content of impurities. The percentage of impurities is not a significant parameter in bioengineering applications, but it should be defined and controlled batch by batch in the preparation of samples for dosimetry applications. The dependence on the preparation batch is still a well-known consideration for those skilled in dosimetry fields.

The signal measured immediately after the irradiation has a contribution of metastable radicals, as demonstrated by the temporal evolution of the EPR signal shown in Fig. 4 for two irradiated samples in the same conditions but from different preparations. The intensity of the peak-to-peak signal reaches a steady state after a few days, when it is reduced by 45% for each sample. It remains substantially stable in the three months after irradiation.

Fig. 4 shows that the time course is of the same type for samples from different preparation batches irradiated under the same conditions (independence from the production batch). This establishes that the processes involved in the transformations of unstable radicals are not influenced by their initial concentration.

Fig. 4 also shows that the number of spin states depends on the preparation, instant by instant. In addition, it shows that the number of stable radicals also depends on the preparation. These observations can be traced to different concentration of CO₂ impurities, representing the site responsible for the formation of the free radical in different preparations.

The temporal behaviour of the EPR signal of HApA is typical of many EPR-sensitive materials used in dosimetry. (7) It is due to excitation of multiple unstable radicals, which evolve into diamagnetic undetectable states in EPR, or towards stable free radical states. The characterization of the temporal behaviour of the EPR signal contributes to the definition of a dosimetric protocol for the material, indicating when it is possible to carry out a reading of a stable signal of interest in dosimetry. This signal is used as the calibration dose. A typical calibration procedure involves the irradiation of a hydrogel sample of the same batch with doses of known value, and the measurement of the corresponding EPR intensities, to obtain the calibration curve of the dosimeter. The unknown absorbed dose by the sample of interest is then obtained for comparison of the measured EPR intensity with the calibration curve.

The dosimetric method for the use of the 3D dosimeter comprises the following steps:
a) Preparation according to the state of the art of a 3D dosimeter consists of a single hydrogel sample having the desired shape and volume, variable linear dimensions from a few mm to several centimetres, average density equal to a fraction of g/cm³, for example that of the lung, as shown in Fig. 1a.
b) Alternatively: preparation of a 3D dosimeter consists of multiple samples of hydrogels, each obtained according to the state of the art with its shape, volume and average density, and subsequently compounded as in Fig. 2a.
c) Irradiation.
d) Subdivision in mass portions of the order of 100 mg, labelled as a function of position.
e) EPR measurement.
f) Reconstruction of the 3D map of the EPR intensity starting from the labellings.
g) Displaying of the 3D map of EPR intensities.
h) Displaying of the 3D map of doses. The EPR signal can be traced to the dose through a usual calibration procedure, for comparison with the EPR intensity obtained by irradiating hydrogels of the same batch with doses of known value.

The step g) can be achieved by eliminating the steps in points d), e) and f), in case of using the EPRI imaging technology (electron paramagnetic resonance imaging). It directly provides the 3D spatial maps of the intensities of the unpaired electron's spin states of the intact (not portioned) 3D dosimeter, and then the maps of doses, through a calibration procedure of the EPRI signal similar to that specified in section h) for the EPR signal.

## Claims

1. 3D dosimeter for the dose verification in radiotherapy and for the quality controls of radiation beams by means of the electron paramagnetic resonance - EPR - reading of the radioinduced signal, in which the dosimetric material consists of a polymeric matrix that incorporates nanometric microporous crystals of inorganic material, **characterized in that** the microporous polymeric matrix that incorporates nanometric microporous crystals of inorganic material is alginate.

2. 3D dosimeter according to claim 1, **characterized in that** the nanometric microporous crystals of inorganic material incorporated in the microporous polymeric matrix consisting of alginate are hydroxyapatite crystals.

3. 3D dosimeter according to claim 1 or 2, **characterized in that** the dosimetric material has a cylindrical shape and linear dimensions, diameter and height, from 1 cm to several cm.

4. 3D dosimeter according to claim 1 or 2 or 3, **characterized in that** the dosimetric material has an average density less than 1 g/cm³.

5. Dosimetric method for use of a 3D dosimeter according to any of the previous claims, **characterized in that** it includes the following steps:
a. Preparation of the 3D dosimeter comprising at least one sample of hydrogel having predetermined shape, size, volume and an average density of less than 1 g/cm³;
b. Irradiation of the at least one sample;
c. EPR measurement;
d. Reconstruction of 3D map of EPR intensities;
e. Displaying the 3D map of EPR intensities;
f. Displaying the 3D map of the irradiation dose.

6. Dosimetric method according to claim 5, wherein the EPR intensity measurement is carried out without imaging, the at least one sample is divided in mass portions in the order of 100 mg, labelled as a function of position; and wherein the reconstruction of 3D map of the EPR intensities and of the radiation doses is made starting from the labelled portions.

7. Dosimetric method according to claim 6, wherein the EPR measurement is made using electron paramagnetic resonance imaging and provides directly the 3D spatial maps of the EPR intensity and of the radiation dose directly from the at least one sample being intact.

## Patentansprüche

1. 3D-Dosimeter zur Dosisüberprüfung bei der Radiotherapie und zur Qualitätskontrolle der Strahlenbündel durch das paramagnetische Elektronenresonanz-Lesen (EPR) des funkinduzierten Signals, in dem das Dosimetrie-Material aus einer Polymermatrix besteht, die nanometrische mikroporöse Kristalle aus anorganischem Material enthält, **dadurch gekennzeichnet, dass** die mikroporöse Polymermatrix, die nanometrische mikroporöse Kristalle aus anorganischem Material enthält, Alginat ist.

2. 3D-Dosimeter nach Anspruch 1, **dadurch gekennzeichnet, dass** die nanometrischen mikroporösen Kristalle aus anorganischem Material, die in der aus Alginat bestehenden mikroporösen Polymermatrix enthalten sind, Hydroxylapatit-Kristalle sind.

3. 3D-Dosimeter nach Anspruch 1 oder 2, **dadurch gekennzeichnet, dass** das Dosimetrie-Material eine zylindrische Form und Längsabmessungen, Durchmesser und Höhe hat, die von 1 cm zu mehrere cm sind.

4. 3D-Dosimeter nach Anspruch 1 oder 2 oder 3, **dadurch gekennzeichnet, dass** das Dosimetrie-Material eine durchschnittliche Dichte hat, die weniger als 1 g/cm³ beträgt.

5. Dosimetrie-Verfahren zur Verwendung eines 3D-Dosimeters nach einem der vorhergehenden Ansprüche, **dadurch gekennzeichnet, dass** es folgende Schritte umfasst:
a. Herstellen des 3D-Dosimeters, das mindestens eine Hydrogel-Probe umfasst, die vorgegebene Form, Größe, Volumen und eine durchschnittliche Dichte von weniger als 1 g/cm³ hat;
b. Bestrahlen der mindestens einen Probe;
c. EPR-Messen;
d. Wiederherstellen der 3D-Mappe der EPR-Intensitäten;
e. Anzeigen der 3D-Mappe der EPR-Intensitäten;
f. Anzeigen der 3D-Mappe der Bestrahlungsdosis.

6. Dosimetrie-Verfahren nach Anspruch 5, in dem die Messung der EPR-Intensität ohne Bildsynthese ausgeführt wird, wobei die mindestens eine Probe in Massenabschnitte in der Größenordnung von 100 mg aufgeteilt ist, die abhängig von der Lage markiert sind; und in dem die Wiederherstellung der 3D-Mappe der EPR-Intensitäten und der Strahlungsdosen ab den markierten Abschnitten ausgeführt wird.

7. Dosimetrie-Verfahren nach Anspruch 6, in dem die EPR-Messung mittels paramagnetischer Elektronenresonanz-Bildsynthese ausgeführt wird und unmittelbar aus der mindestens einen intakten Probe die räumlichen 3D-Mappen der EPR-Intensität und der Strahlungsdosis bereitstellt.

## Revendications

1. Dosimètre 3D pour la vérification de dose dans la radiothérapie et pour les contrôles de qualité de faisceaux de rayonnement au moyen de lecture de résonance paramagnétique électronique RPE du signal radio-induit, dans lequel la matière dosimétrique consiste en une matrice polymérique qui incorpore des cristaux microporeux nanométriques en matière inorganique, **caractérisé en ce que** la matrice polymérique microporose qui incorpore des cristaux microporeux nanométriques en matière inorganique est de l'alginate.

2. Dosimètre 3D selon la revendication 1, **caractérisé en ce que** les cristaux microporeux nanométriques en matière inorganique incorporés dans la matrice polymérique microporose consistant en alginate sont des cristaux d'hydroxyapatite.

3. Dosimètre 3D selon la revendication 1 ou 2, **caractérisé en ce que** la matière dosimétrique a une forme cylindrique et des dimensions linéaires, un diamètre et une hauteur, de 1 cm à plusieurs cm.

4. Dosimètre 3D selon la revendication 1 ou 2 ou 3, **caractérisé en ce que** la matière dosimétrique a une densité moyenne inférieure à 1 g/cm³.

5. Procédé de dosimétrie d'utilisation d'un dosimètre 3D selon l'une quelconque des revendications précédentes, **caractérisé en ce qu'**il inclut les étapes suivantes :
a. préparation du dosimètre 3D comprenant au moins un échantillon d'hydrogel ayant une forme, une dimension et un volume prédéterminés et une densité moyenne inférieure à 1 g/cm³ ;
b. irradiation de l'au moins un échantillon ;
c. mesure RPE ;
d. reconstruction de plan 3D d'intensités RPE ;
e. affichage du plan 3D d'intensités RPE ;
f. affichage du plan 3D de la dose d'irradiation.

6. Procédé de dosimétrie selon la revendication 5, dans lequel la mesure d'intensité RPE est effectuée sans formation d'image, l'au moins un échantillon étant divisé en parties de masse de l'ordre de 100 mg, marquées en fonction de la position ; et dans lequel la reconstruction de plan 3D des intensités RPE et des doses de rayonnement est effectué depuis les portions marquées.

7. Procédé de dosimétrie selon la revendication 6, dans lequel la mesure RPE est effectuée en utilisant la formation d'image par résonance paramagnétique électronique et fournit directement le plan 3D spatial de l'intensité RPE et de la dose de rayonnement directement à partir de l'au moins un échantillon étant intact.
